# EUROPÄISCHE PATENTANMELDUNG

(11) **EP 1 469 078 A1**
(43) Veröffentlichungstag der Anmeldung: **20.10.2004**
(21) Anmeldenummer: 04008644.9
(22) Anmeldetag: 08.04.2004
(51) Int. Cl.: C12P 1/02, C12P 7/26, C12R 1/645, C12N 1/16, C12N 1/38

(54) **Verfahren zur Herstellung von Sporidiobolus ruineniae Stämmen mit verbesserter Coenzym Q10-Produktion**

(30) Priorität: 17.04.2003 DE 10317877
(71) Anmelder: Consortium für elektrochemische Industrie GmbH, 81379 München (DE)
(72) Erfinder: Pfaller, Rupert, Dr., 80639 München (DE); Leonhartsberger, Susanne, Dr., 80634 München (DE)
(74) Vertreter: Potten, Holger

(57) **Zusammenfassung**

Verfahren zur Herstellung eines mutagenisierten Sporidiobolus ruineniae Stammes mit einer Q10-Produktivität von größer 1,38 mg Q10/g Trockenbiomasse dadurch gekennzeichnet, dass
a) ein Sporidiobolus ruineniae Stamm einer Genveränderung durch Mutagenese ausgesetzt wird und
b) der mutagenisierte Sporidiobolus ruineniae Stamm einer Selektion unterworfen wird, wobei der mutagenisierte Sporidiobolus ruineniae Stamm unter Bedingungen, die ein Wachstum des in a) eingesetzten Sporidiobolus ruineniae Stammes hemmen, kultiviert wird, wobei die Bedingungen so gewählt sind, dass der mutagenisierte Stamm durch eine im Vergleich zu dem in a) eingesetzten Sporidiobolus ruineniae Stamm erhöhte Q10-Produktion die Wachstumshemmung uberwindet, in einem Fermentationsmedium wächst und
c) aus dem Fermentationsmedium isoliert wird.

## Beschreibung

Die Erfindung betrifft ein Verfahren zur Herstellung von Sporidiobolus ruineniae Stammen mit verbesserter Coenzym Q10-Produktion, so erhältliche Stämme der Art Sporidiobolus ruineniae (S. ruineniae) sowie die Verwendung der Stämme zur Produktion von Ubichinon-10 (Coenzym Q10, in der Folge Q10 benannt).

Coenzym Q10 ist eine Verbindung aus der Familie der Ubichinone mit der allgemeinen Formel:

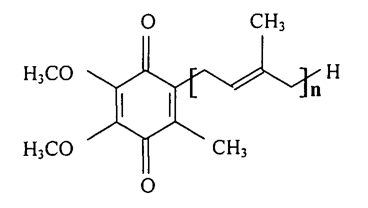

Die chemische Struktur von Q10 wird nach der o.g. Formel beschrieben aus einem ringsubstituierten Chinonderivat und einer hydrophoben Kette von n Isopreneinheiten der allg. Formel -CH₂-CH=C(CH₃)-CH₂-. Die Zahl n der Isopreneinheiten ist spezifisch für das Ubichinon eines gegebenen Organismus.

Beim Q10 handelt es sich um ein Ubichinon nach der o.g. allg. Formel, wobei die Zahl n der Isopreneinheiten in Q10 n = 10 beträgt (entsprechend einer Isoprenkette von 50 C-Atomen). Q10 ist in natürlichen Organismen weit verbreitet, v.a. im Menschen, anderen höheren Organismen aus der Tier- und Pflanzenwelt, aber auch in verschiedenen Arten von Bakterien, Pilzen oder Hefen. Andere bekannte Ubichinone sind Q6 (Bäckerhefe), Q8 (E. coli), Q9 (Rhodobacter capsulatus).

Die chemische Struktur des Q10 bedingt seine physiologischchemische Funktion sowie die Lokalisierung innerhalb einer Zelle. Die chinoide Struktur besitzt die biochemische Effektorfunktion und ermöglicht die reversible Elektronenaufnahme (Reduktion durch zwei Elektronen, Hydrochinonform) und -abgabe (Oxidation, Chinonform). Als Zwischenstufe ist auch die Semichinonform (Aufnahme, bzw. Abgabe von nur einem Elektron) bekannt. Die hydrophobe Isoprenkette bewirkt die Lokalisierung des Q10 in Zellmembranen.

Zu den bekannten biochemischen und physiologischen Funktionen von Q10 gehören (zusammengefasst in dem Übersichtsartikel "Biochemical Functions of Coenzyme Q10". F. L. Crane, J. of the Am. College of Nutrition (2001) 20: 591-598):
- Elektronenüberträger in der Atmungskette (Energieumwandlung).
- Schutz vor ox. Schädigung in Zellmembranen (Antioxidansfunktion und Regenerierung anderer Antioxidanzien).
- Stimulierung des Zellwachstums und Hemmung des Zelltods.

Aufgrund der biochemischen und physiologischen Funktion findet Q10 Anwendung in verschiedenen Bereichen wie Pharma, Lebensmittel und Kosmetik.

Bekannte Einsatzgebiete für Q10 im pharmazeutischen Bereich sind Herzinsuffizienz und andere cardiovaskuläre Krankheiten. Im Lebensmittelbereich wird Q10 eingesetzt als Nahrungsergänzungsmittel in Vitaminpräparaten.
In der Kosmetik findet Q10 neuerdings Verwendung als Zusatzstoff in Hautcremes und auch der Einsatz in Zahnpflegemitteln wird getestet.

Es sind keine kostengünstigen chemischen Syntheseverfahren für Q10 bekannt. Die Herstellung von Q10 erfolgt durch Extraktion aus natürlich vorkommenden Quellen. Generell ist der Q10-Gehalt in natürlich vorkommenden Quellen so niedrig, dass die Q10-Gewinnung durch Extraktion unwirtschaftlich ist. Es gibt nur wenige natürliche Quellen, deren Q10-Gehalt genügend hoch ist (z. B. einige wenige Mikroorganismen oder pflanzliche Öle). Daher ist trotz steigenden Bedarfs der Preis für Q10 immer noch sehr hoch.

Verschiedene Mikroorganismen mit vergleichsweise hoher Q10-Produktion wurden beschrieben. Zu den bakteriellen Produzenten gehören unter anderem Stamme aus den Gattungen Agrobakterium, Rhodobakter oder Hyphomonas. Es wurde eine zum Teil hohe Q10-Produktivität beschrieben, eine kommerzielle Nutzung ist jedoch unbekannt.

Bekannte Hefestämme mit Q10-Produktion sind Vertreter beispielsweise aus den Gattungen Sporobolomyces, Rhodotorula, Oosporidium und Sporidiobolus. Speziell Stämme der Art Sporidiobolus ruineniae wurden frühzeitig bereits im Jahr 1978 als gute Produzenten von Q10 erkannt (US 4070244) mit einer Q10-Produktivität von 0,92 mg/g Biomasse, was mit zur höchsten Produktivität unter den bekannten Q10-produzierenden Hefestämmen gehört.

Trotz der früh entdeckten Eigenschaft von Sporidiobolus ruineniae als guter Q10-Produzent sind seitdem keine Verbesserungen dokumentiert, die zu einer kommerziellen Nutzung von Sporidiobolus ruineniae für die Q10-Herstellung geführt hätten. Speziell sind keine Verfahren zur Isolierung von S. ruineniae Stämmen mit verbesserter Q10-Produktion sowie keine Weiterentwicklung des fermentativen Verfahrens zur Q10-Herstellung bekannt, was auf Schwierigkeiten bei der Weiterentwicklung von Sporidiobolus ruineniae als Produktionssystem zur Q10-Herstellung schließen lässt. Diese Schwierigkeiten werden mit der vorliegenden Erfindung überwunden.

Die Erfindung betrifft ein Verfahren zur Herstellung eines mutagenisierten Sporidiobolus ruineniae Stammes mit einer Q10-Produktivität von größer 1,38 mg Q10/g Trockenbiomasse dadurch gekennzeichnet, dass
a) ein Sporidiobolus ruineniae Stamm einer Genveränderung durch Mutagenese ausgesetzt wird und
b) der mutagenisierte Sporidiobolus ruineniae Stamm einer Selektion unterworfen wird, wobei der mutagenisierte Sporidiobolus ruineniae Stamm unter Bedingungen, die ein Wachstum des in a) eingesetzten Sporidiobolus ruineniae Stammes hemmen, kultiviert wird, wobei die Bedingungen so gewählt sind, dass der mutagenisierte Stamm durch eine im Vergleich zu dem in a) eingesetzten Sporidiobolus ruineniae Stamm erhöhte Q10-Produktion die Wachstumshemmung überwindet, in einem Fermentationsmedium wächst und
c) aus dem Fermentationsmedium isoliert wird.

An der Biosynthese von Q10 in Mikroorganismen sind zwei Stoffwechselwege beteiligt, nämlich der Syntheseweg zu aromatischen Aminosäuren und der Isoprenoidstoffwechsel. Ein detaillierter Überblick über die Biosynthese der Ubichinone findet sich in "Ubiquinone Biosynthesis in Microorganisms". R. Meganathan, FEMS Microbiology Letters (2001) 203: 131-139.

Durch Kenntnis der biochemischen Funktion und des Biosyntheseweges können in erfinderischer Weise Stämme mit verbesserter Q10-Produktion isoliert werden. Das Prinzip des erfindungsgemäßen Verfahrens (Screenings) beruht auf einem selektiven Zellwachstum: S. ruineniae Zellen werden unter Bedingungen kultiviert, die normalerweise das Zellwachstum unterdrücken. Die wachstumshemmenden Bedingungen sind so gewählt, dass die Zellen durch erhöhte Q10-Produktion die Wachstumshemmung überwinden und wieder wachsen können. Eine erhöhte Q10-Produktion des verbesserten Stammes ist im Anschluss an das Screening auch in Abwesenheit des wachstumshemmenden Stoffes zu beobachten.

Die erhöhte Q10-Produktion kann dabei auf verschiedene Weise bewerkstelligt werden, beruht aber immer auf einer genetischen Veränderung des S. ruineniae Stammes. Die genetische Veränderung, die zu einer erhöhten Q10-Produktion in S. ruineniae führt, erfolgt bevorzugt durch Genveränderungen aufgrund Einwirkung mutagener Substanzen oder hochenergetischer Strahlung auf das S. ruineniae Genom (Mutagenese), durch Fusionierung verschiedener S. ruineniae Zellen und dadurch hervorgerufener genetischer Rekombination (sog. "Genome Shuffling"), oder durch gezielten Eingriff in den Q10 Biosyntheseweg mit gentechnischen Methoden. Mutanten mit veränderten Eigenschaften wie z.B. erhöhter Q10-Produktion können durch geeignete Selektionsbedingungen identifiziert werden.

Zur Mutagenese geeignete Substanzen sind N-Methyl-N'-Nitro-N-Nitrosoguanidin (NG), Ethylmethansulfonat (EMS), Dimethylsulfat (DMS), und alle anderen vergleichbaren mutagenen Substanzen. Erbgutschädigende Strahlung ist energiereiches, kurzwelliges UV-Licht, Röntgenstrahlung oder radioaktive Strahlung.

Es sind jedoch auch jede andere Art erbgutschädigender Substanzen bzw. Strahlungen geeignet, eine ungerichtete Veränderung des Erbguts zu bewirken mit dem Effekt, dass die Q10 Produktion in einer Mutante von S. ruineniae gesteigert wird.

Bevorzugt zur Mutagenese verwendet wird NG, EMS und UV-Strahlung. Besonders bevorzugt zur Mutagenese verwendet wird NG und UV-Strahlung.

Bei der Mutagenese wird das Mutagen in einer Menge eingesetzt, die vorzugsweise eine Abtötungsrate der mutagenisierten S. ruineniae Zellen von 30 bis 99 % vor der anschließenden Selektion bewirkt. Die bevorzugte Abtötungsrate bei der Mutagenese ist 40 bis 90 % der S. ruineniae Zellen. Besonders bevorzugt ist eine Abtötungsrate von 50 bis 90 % der S. ruineniae Zellen. Dies wird beispielsweise durch eine NG-Konzentration von 0,01 - 0,06 mg/ml erreicht.

Verschiedene Methoden können eingesetzt werden, um auf erfindungsgemäße Weise durch selektives Zellwachstum Mutanten mit verbesserter Q10-Produktion zu isolieren.

Bedingungen selektiven Zellwachstums können auf der Ebene des Q10-Biosyntheseweges erzeugt werden. Durch die Selektionsbedingungen werden erfindungsgemäß einzelne Biosyntheseschritte des Q10-Biosyntheseweges gehemmt. Dazu gehören alle Schritte des Biosyntheseweges der aromatischen Aminosäuren zur 4-Hydroxy-Benzoesäure und schließlich zum Tyrosin und alle Schritte des Biosyntheseweges zur Isoprenseitenkette.

Bevorzugt ist die Hemmung des Biosyntheseweges aromatischer Aminosäuren auf der Stufe von Shikimat zu Chorismat durch einen kompetitiven Inhibitor dieses biochemischen Syntheseschrittes wie N-Phosphonomethylglycin (bekannt auch unter den Namen Glyphosat bzw. Round Up).

Bevorzugt ist auch die kompetitive Hemmung der Synthese der Isoprenseitenkette auf der Ebene der Synthese der Mevalonsäure. Besonders geeignet sind kompetitive Hemmstoffe des Enzyms HMG-CoA Reduktase (Sog. Statine, bzw. Cholesterinsenker). Geeignete selektive Hemmstoffe sind Statine wie Lovastatin, Cerivastatin, Atorvastatin und Compactin (Mevastatin), jeweils in ihrer freien oder ihrer Lactonform bzw. jede Art von Hemmstoffen, die durch Hemmung der HMG-CoA-Reduktase wirken, beispielsweise Mevalonat, das die Synthese der HMG-CoA-Reduktase beeinflusst (Dimster-Denk, 1994). Besonders bevorzugt sind Lovastatin und Cerivastatin.

Weitere Reaktionsschritte aus den zu Isoprenoiden führenden Biosynthesewegen, die sich für die Selektion von S. ruineniae Stämmen mit verbesserter Q10-Produktion eignen, aber nicht auf diese beschränkt sind, sind die Reaktionsschritte, die zum Geranylpyrophosphat, zum Farnesylpyrophosphat, zum Squalen oder beispielsweise zum Phytoen führen.

Die Biosynthese des Q10-Chinongerüstes beinhaltet drei Methylierungsschritte, wobei S-Adenosylmethionin als Donor der Methylgruppe fungiert. Stämme mit einem erhöhten Spiegel an S-Adenosylmethionin, die zur erhöhten Q10-Produktion befähigt sind, können beispielsweise mit dem selektiven Agens Ethionin, einem Methioninanalogon isoliert werden.

Ein weiteres bevorzugtes erfindungsgemäßes Verfahren zu Selektion von S. ruineniae Stammen mit verbesserter Q10-Produktion erfolgt über die biochemische Funktion des Q10. Aufgrund der Antioxidanswirkung von Q10 eignen sich alle Bedingungen für ein erfindungsgemäßes Selektionsverfahren, die oxidativen Stress erzeugen. Agentien, die oxidativen Stress erzeugen, sind z.B. aktivierte Sauerstoffspezies. Dazu zählen Wasserstoffperoxid einschließlich aller möglichen, vom Wasserstoffperoxid abgeleiteten Peroxide sowie alle in situ aus der Reaktion von Wasserstoffperoxid mit anderen Substanzen erzeugten reaktiven, oxidativen Spezies. Ein Beispiel dafür ist die Kombination aus Wasserstoffperoxid und Linolensäure bzw. ungesättigten Fettsäuren im allgemeinen. Die Erzeugung reaktiver Lipidperoxide aus einfach oder mehrfach ungesättigten Fettsäuren wird in Do et al., Proc. Natl. Acad. Sci. USA (1996) 93: 7534-7539 beschrieben. Zu den reaktiven Sauerstoffspezies zählen weiterhin das Superoxidradikal bzw. Verbindungen wie Paraquat (1,1`-Dimethyl-4,4`bipyridiniumdichlorid, auch Methylviologen oder Gramoxone genannt), die zur Erzeugung von Superoxidradikalen geeignet sind. Außerdem gehören das Hydroxylradikal und Verbindungen, die zur Bildung von Hydroxylradikalen geeignet sind, in diese Klasse. Des weiteren kann ein oxidativer Stress auf die Zellen ausgeübt werden, dadurch dass sie einem Hitzeschock unterzogen werden.

Bevorzugte Agentien für ein Selektionsverfahren durch oxidativen Stress sind Wasserstoffperoxid, davon abgeleitete Peroxide sowie die aus der Kombination von Wasserstoffperoxid mit anderen Substanzen erzeugten reaktiven, oxidativen Spezies und Verbindungen wie Paraquat, die zur Bildung des Superoxidradikals befähigt sind, außerdem ungesättigte Fettsäuren wie Linolensäure. Besonders bevorzugte Agentien für ein Selektionsverfahren durch oxidativen Stress sind Wasserstoffperoxid, Paraquat und Linolensäure bzw. andere einfach oder mehrfach ungesättigte Fettsäuren oder eine Kombination dieser Agenzien.

Als zweite biochemische Funktion des Q10, die sich als weiteres bevorzugtes erfindungsgemäßes Verfahren zur Selektion von S. ruineniae Stämmen mit verbesserter Q10-Produktion eignet, dient die Funktion von Q10 als Elektronenüberträger in der Atmungskette. Aufgrund dieser Funktion eignen sich Inhibitoren, welche die Funktion der Atmungskette beeinträchtigen können, zur Selektion von Zellen mit verbesserter Q10 Produktion. Q10 dient in der Atmungskette als Elektronenübertrager zwischen den Komplexen I und II auf der einen Seite (Elektronenaufnahme) und dem Komplex III auf der anderen Seite (Elektronenabgabe). Die Funktion von Q10 in der Atmungskette ist zusammengefasst in dem Übersichtsartikel "Biochemical Functions of Coenzyme Q10". F. L. Crane, J. of the Am. College of Nutrition (2001) 20: 591-598.

Verschiedene Verbindungen sind bekannt, die als Inhibitoren die Funktion der Atmungskette beeinträchtigen können (siehe "Mitochondria" von A. Tzagoloff, S. 92-95, 1982 Plenum Press New Y-ork, ISBN 0-306-40778-7). Neben generellen Inhibitoren wie z. B. Cyanid oder Kohlenmonoxid eignen sich vor allem Strukturanaloga von Q10 für das erfindungsgemäße Selektionsverfahren zur Isolierung von S. ruineniae Stämmen mit verbesserter Q10-Produktion. Beispiele geeigneter Inhibitoren der Atmungskette sind Verbindungen wie Antimycine, speziell Antimycin A, Rotenon, Amobarbital, Capsaicin, MPP+, Myxothiazol, Mucidin, Menadion oder Piericidin. Bevorzugte Inhibitoren sind Menadion, Rotenon, Antimycin A und Piericidin. Besonders bevorzugte Inhibitoren sind Menadion, Antimycin A und Piericidin.

Die genannten selektiven Agentien zur Isolierung von S. ruineniae Stämmen mit verbesserter Q10-Produktion können in allen denkbaren Konzentrationen sowie auch in jeder möglichen Kombination miteinander eingesetzt werden.

Die selektiven Agentien werden bevorzugt in einer Konzentration von 0,005 % (w/v) bis 1 % (w/v) (Paraquat, Wasserstoffperoxid), 5 mM bis 500 mM (Linolensäure), 0,001 mM bis 1 mM (Antimycin A, Piericidin A, Menadion) eingesetzt.

Die selektiven Agentien werden besonders bevorzugt in einer Konzentration von 0,01 % (w/v) bis 0,3 % (w/v) (Paraquat), 20 mM bis 300 mM (Linolensäure), 0,01 mM bis 0,5 mM (Antimycin A, Piericidin A, Menadion) eingesetzt.

Bevorzugte Kombinationen selektiver Agentien, in den oben angegebenen, bevorzugten Konzentrationsbereichen sind Wasserstoffperoxid + Paraquat, Wasserstoffperoxid + Linolensäure, Paraquat + Linolensäure, Paraquat + Linolensäure + Piericidin A, Paraquat + Antimycin A sowie Paraquat + Piericidin.

Besonders bevorzugt, in den oben angegebenen besonders bevorzugten Konzentrationsbereichen, sind Kombinationen von Wasserstoffperoxid und Paraquat, von Wasserstoffperoxid und Linolensäure, von Paraquat und Linolensäure, sowie von Paraquat und Linolensäure und Piericidin A.

Mutanten von S. ruineniae mit verbesserter Q10-Produktion werden nach dem folgenden, erfindungsgemäßen Verfahren isoliert. S. ruineniae wird auf die bereits beschriebene Weise mutagenisiert. Mutanten, die unter den erfindungsgemäßen, selektiven Bedingungen wachsen, werden gepickt und weiter kultiviert. Vergleichende Tests zur Auswahl von Mutanten mit verbesserter Q10-Produktion werden durch Anzucht im Schüttelkolben vorgenommen.

Isolierte Mutanten werden submers in einem Wachstumsmedium kultiviert, um Biomasse zu erzeugen. Die Biomasse wird isoliert und daraus in an sich bekannter Weise Q10 extrahiert. Die Q10 Menge wird quantitativ bestimmt durch Hochdruck-Flüssigchromatographie (HPLC), wobei jede quantitative Methode zur Q10-Bestimmung geeignet ist.

Aus dem Vergleich der Q10-Produktion der verschiedenen Mutanten mit dem Ausgangsstamm wird die Mutante mit der höchsten Q10-Produktion ausgewählt und in eine neue Runde der genannten Mutagenese und Selektion eingeschleust. Die Bedingungen der neuerlichen Mutagenese und Selektion werden dabei nach den oben definierten, bevorzugten Bedingungen ausgewählt. Mit dieser Methode gelingt es, erfindungsgemäße Stämme von S. ruineniae zu isolieren, die für die biotechnologische Produktion von Q10 geeignet sind und die gegenüber dem Stand der Technik deutlich verbessert sind. Solche Stämme werden vorzugsweise durch 1 bis 10 Runden Mutagenese und Selektion, besonders bevorzugt durch 2 bis 6 Runden Mutagenese und Selektion erhalten.

Das erfindungsgemäße Verfahren ermöglicht somit die Gewinnung deutlich verbesserter Q10-produzierender Stamme von S. ruineniae. Deutlich verbesserte Q10-produzierende Stämme von S. ruineniae sind definiert als solche, deren Q10-Produktivität gegenüber dem in US 4070244 offenbarten Stand der Technik von 0,92 mg Q10/g Biomasse um mindestens 50 % gesteigert ist. Diese Stämme besitzen somit eine Q10-Produktivität von mehr als 1,38 mg Q10/g Biomasse. Ein S. ruineniae Stamm mit einer Q10-Produktivität von mehr als 1,38 mg Q10/g Biomasse, welcher durch das erfindungsgemäße Verfahren der Mutagenese und Selektion erhältlich ist, ist somit ebenfalls Gegenstand der Erfindung.

Für die Stammverbesserung nach dem beschriebenen Verfahren verwendet wird der Stamm Sporidiobolus ruineniae Sr-1 (hinterlegt gemäß Budapester Vertrag unter der Nummer DSM 15553 bei der Deutschen Sammlung für Mikroorganismen und Zellkulturen GmbH, D-38142 Braunschweig).

Eine weitere Aufgabe der vorliegenden Erfindung war es, ein verbessertes Verfahren zur Q10-Produktion zur Verfügung zu stellen. Diese Aufgabe wird gelöst durch ein Verfahren, dadurch gekennzeichnet, dass Zellen eines erfindungsgemäß verbesserten Q10-produzierenden Stammes von S. ruineniae in einem Anzuchtmedium kultiviert werden und die Zellen nach einer Wachstumszeit von 70 bis 150 h isoliert werden. Zur Produktion von Q10 werden die ausgewählten Stämme in einem Anzuchtsmedium kultiviert, um Biomasse zu erzeugen. Die Anzucht erfolgt in einer dem Fachmann geläufigen Weise. Dazu kann die Anzucht in Schüttelkolben (Labormassstab) erfolgen oder aber auch durch Fermentation.

Dem Fachmann geläufige Anzuchtsmedien sind in der Literatur beschrieben (US 4070244) und bestehen typischerweise aus einer Kohlenstoffquelle (C-Quelle), einer Stickstoffquelle (N-Quelle) sowie Zusätzen wie Vitaminen, Salzen und Spurenelementen, durch die das Zellwachstum optimiert wird. C-Quellen sind solche, die von S. ruineniae zur Biomassebildung genützt werden können. Dazu gehören Glucose, Dextrose, Maltose, Acetat (Essigsäure), Glycerin und Ethanol. Verwertbare C-Quellen bei der Anzucht von Stämmen der Gattung Sporidiobolus sind im Stand der Technik bekannt. Einen Überblick gibt z.B. die Internetadresse (www.cbs.knaw.nl/cbshome.html) der holländischen Stammsammlung "Centraalbureau voor Schimmelcultures", CBS.

Bevorzugte C-Quellen zur Anzucht von Stämmen der Gattung Sporidiobolus sind Glucose, Stärke, Maltodextrin, Glycerin, Maltose, Essigsäure und ihre Salze, Saccharose, Melasse, Zitronensäure und ihre Salze.

N-Quellen sind solche, die von Sporidiobolus ruineniae zur Biomassebildung genützt werden können. Dazu gehören Ammoniak, gasförmig oder in wässriger Lösung als NH₄OH oder aber auch dessen Salze wie z. B. Ammoniumsulfat, Ammoniumchlorid oder Ammoniumnitrat. Des weiteren sind als N-Quelle geeignet die bekannten Nitratsalze wie z. B. KNO₃, NaNO₃, Ammoniumnitrat, Ca(NO₃)₂, Mg(NO₃)₂ sowie andere N-Quellen wie Harnstoff. Zu den N-Quellen gehören auch komplexe Aminosäuregemische wie Hefeextrakt (darunter auch technische Hefeextrakte wie z. B. HY Yest444 der Firma Quest), Proteose Pepton, Malzextrakt, Sojapepton, Casaminosäuren, Maisquellwasser (Corn Steep Liquor) sowie auch NZ-Amine und Yeast Nitrogen Base.

Die Anzucht kann erfolgen im sog. Batch Modus, wobei das Anzuchtsmedium mit einer Starterkultur von Sporidiobolus ruineniae inokuliert wird und dann das Zellwachstum ohne weitere Fütterung von Nährstoffquellen erfolgt.

Die Anzucht kann auch erfolgen im sog. Fed-Batch Modus, wobei nach einer anfänglichen Phase des Wachstums im Batch Modus zusätzlich Nährstoffquellen zugefüttert werden (Feed), um deren Verbrauch auszugleichen. Der Feed kann bestehen aus der C-Quelle, der N-Quelle oder aus einem Gemisch der beiden. Zusätzlich können auch andere Medienbestandteile sowie spezifisch die Q10-Produktion steigernde Zusätze dem Feed zugesetzt sein.

Bevorzugt ist die Anzucht nach dem Fed-Batch Modus.

Bevorzugte C-Quellen im Feed sind Glycerin, Glucose, Saccharose, Melasse, Maltose oder Acetat.

Bevorzugte N-Quellen im Feed sind Ammoniak, gasförmig oder in wässriger Lösung als NH₄OH und dessen Salze Ammoniumsulfat und Ammoniumchlorid, weiterhin KNO₃, NaNO₃ und Ammoniumnitrat, Hefeextrakt, HY Yest444, Proteose Pepton, Malzextrakt, Sojapepton, Casaminosäuren, Maisquellwasser (Corn Steep Liquor) sowie auch NZ-Amine und Yeast Nitrogen Base.

Besonders bevorzugte N-Quellen im Feed sind Ammoniak bzw. Ammoniumsalze, Hefeextrakt, Sojapepton, Malzextrakt oder Corn Steep liquor.

Dem Anzuchtsmedium können auch solche Komponenten zugesetzt sein, die spezifisch für eine Steigerung der Q10-Produktion geeignet sind (sog. Induktoren). Dazu zählen Verbindungen wie p-Hydroxy-Benzoesäure sowie dessen Salze und Ester (US 4070244), Isopentenol (US 4220719), Tyrosin und seine biochemischen Vorläufer wie Shikimisäure oder Chorisminsäure und ungesättigte Fettsäuren wie Ölsäure (R.A. Hagerman et al., Free Radical Research (2002) 36: 485-490).

Die Anzucht erfolgt unter pH- und Temperaturbedingungen, welche das Wachstum und die Q10-Produktion von Sporidiobolus ruineniae begünstigen. Der nützliche pH-Bereich reicht von pH 4 bis pH 10. Bevorzugt ist ein pH-Bereich von pH 4 bis pH 9. Besonders bevorzugt ist ein pH-Bereich von pH 5,0 bis pH 8,5.

Der bevorzugte Temperaturbereich für das Wachstum von S. ruineniae beträgt 20°C bis 40°C. Besonders bevorzugt ist der Temperaturbereich von 25°C bis 35°C.

Für das Wachstum von S. ruineniae ist eine ausreichende Versorgung mit Sauerstoff erforderlich. Die Sauerstoffversorgung wird vorzugsweise durch Eintrag von Pressluft oder reinem Sauerstoff gewährleistet.

Die Dauer des Wachstums von S. ruineniae zur Q10-Produktion beträgt zwischen 10 h und 200 h. Bevorzugt ist eine Wachstumszeit von 30 h bis 120 h. Besonders bevorzugt ist eine Wachstumszeit von 40 h bis 100 h.

S. ruineniae Zellen, die durch das oben beschriebene Verfahren zur Anzucht gewonnen werden, enthalten das Q10-Produkt. Das Q10 kann aus diesen Zellen extrahiert und isoliert werden. Die Zellen können aber auch direkt für die weitere Verwendung aufbereitet werden, ohne dass das Q10 daraus isoliert wird.

Methoden zur Isolierung von Q10 sind bekannt (US 4070244) und beinhalten einen Extraktionsschritt, bei dem Q10 aus der Zellmembran herausgelöst werden muss. Dieser Schritt beinhaltet die Zerstörung der Zellstruktur, welche entweder mechanisch (durch mahlen) oder chemisch mit Lösungsmitteln erfolgen kann. Das Q10 wird dann in ein org. Lösungsmittel extrahiert und von verunreinigenden Bestandteilen gereinigt. Dies kann erfolgen durch Fällungsmethoden, chromatographische Verfahren oder auch durch spezifische Komplexbildung (z. B. Cyclodextrine). Verschiedene analytische Methoden zur Identifizierung des isolierten Q10 sind verfügbar, zu denen unter anderen Massenspektroskopie, NMR, UV/Vis-Absorptionsspektroskopie, HPLC oder auch eine Kombination daraus gehören.

Bevorzugte Methode zur Analytik von Q10 ist HPLC.

Methoden zur Aufbereitung der Sporidiobolus ruineniae Zellen aus der Anzucht zur direkten Weiterverwendung ohne vorherige Reinigung des darin enthaltenen Q10 sind Gefriertrocknung, Sprühtrocknung, Hitzetrocknung sowie Mahlen und Sieben der erhaltenen, getrockneten Biomasse. Unter geeigneten Bedingungen kann das so aufbereitete Q10 direkt für eine Anwendung weiterverwendet werden.

Die folgenden Beispiele dienen der weiteren Erläuterung der Erfindung.

### 1. Beispiel: Isolierung von Q10.

### Q10-Isolierung ohne mechanischen Zellaufschluss von S. ruineniae:

Die Methode wurde beim Screening von Mutanten mit verbesserter Q10-Produktion eingesetzt. 50 ml YPGC-Medium (1 % Hefeextract, 2 % Pepton, 5 % Glycerin, 2 % Mais Quellwasser (Corn Steep Liquor)) wurden mit Sporodiobolus ruineniae Sr-1 (DSM 15553) beimpft und 120 h auf einem Orbitalschüttler (Infors, 140 rpm) bei 28°C bebrütet. Der Ansatz wurde in ein 50 ml Zentrifugenröhrchen (Falconröhrchen) überführt, die S. ruineniae Zellen abzentrifugiert (10 min 3000 rpm, Heraeus Megafuge 1.0R), in 30 ml vollentsalztem Wasser gewaschen, in 5 ml vollentsalztem Wasser resuspendiert und in 50 ml Rundkolben überführt. Die Zellsuspension wurde anschließend in einer Ethanol/Trockeneis-Mischung möglichst gleichmäßig eingefroren und lyophilisiert (Christ Gefriertrockner "alpha 2-4"). 200 mg Trockenmasse wurde in ein 12 ml Glasröhrchen eingewogen, mit 10 ml Ethanol versetzt und mit gasförmigem Stickstoff überlagert. Zur Extraktion wurde über Nacht bei 40 °C waagrecht geschüttelt. Danach wurde die Suspension abzentrifugiert, der Überstand gesammelt und mit Stickstoff überlagert. In einer zweiten Extraktion wurde der Niederschlag in 8-10 ml Ethanol aufgenommen, mit Stickstoff überlagert, gut gemischt und 2 Stunden bei 40 °C waagrecht geschüttelt. In einer dritten Extraktion wurde der Vorgang wiederholt und die Suspension 1 Stunde bei 40 °C waagrecht geschüttelt. Die Überstände der Extraktionen 1-3 wurden vereinigt, mit 5 ml vollentsalztem Wasser und 10 ml n-Heptan gemischt und zur Phasentrennung abzentrifugiert. Die gefärbte, obere n-Heptanphase wurde in einen 50 ml Rundkolben pipettiert, die untere Phase nochmals mit 10 ml n-Heptan ausgeschüttelt und zur Phasentrennung zentrifugiert. Die n-Heptan Phasen wurden vereinigt und das Lösungsmittel über einen Rotationsverdampfer entfernt. Es verblieb ein rötlicher Rückstand, der in 1 ml THF aufgenommen und für die quantitative Bestimmung durch kalibrierte HPLC verwendet wurde.

### Q10-Isolierung mit mechanischem Zellaufschluss von S. ruineniae:

Die Methode wurde zur Q10-Bestimmung von Fermenterproben eingesetzt. 25 ml aus einer Fermentation entnommene Zellsuspension (siehe 6. und 7. Beispiel) wurde in ein 50 ml Zentrifugenröhrchen (Falconröhrchen) überführt, die S. ruineniae Zellen abzentrifugiert (10 min 3000 rpm, Heraeus Megafuge 1.0R) und das Zellpellet einmal mit Wasser gewaschen. Das Zellpellet wurde in 10 ml Wasser aufgeschlämmt und quantitativ in einen tarierten 50 ml Glasrundkolben überführt. Die Zellsuspension wurde gefroren, über Nacht gefriergetrocknet (Christ Gefriertrockner "alpha 2-4") und anschließend durch Auswiegen die Biotrockenmasse bestimmt. Darauf folgte ein mechanischer Zellaufschluss mit einem sog. Dismembrator (Braun, Melsungen). Dazu wurden 300 mg der Trockenbiomasse in eine für den Dismembrator passenden 3 ml Teflonkammer eingewogen, eine Stahlkugel (Durchmesser 5 mm) dazugegeben, die Teflonkammer verschlossen und in flüssigem Stickstoff gefroren. Anschließend wurde die tiefgefrorene Teflonkammer in den Dismembrator eingespannt und für 2 min bei einer Schwingzahl von 2000/min geschüttelt. 200 mg der auf diese Weise mechanisch aufgeschlossenen Trockenbiomasse wurden in ein 12 ml Glasröhrchen eingewogen, mit 8 ml n-Heptan versetzt und über Nacht bei 40°C geschüttelt. Der Ansatz wurde dann zentrifugiert (10 min 3000 rpm, Heraeus Zentrifuge) und die organische Phase in einen 100 ml Glasrundkolben überführt. Die Extraktion mit n-Heptan wurde noch zweimal wiederholt (je 2 h schütteln bei 40°C). Die n-Heptan Phasen wurden vereinigt und das Lösungsmittel über einen Rotationsverdampfer entfernt. Es verblieb ein rötlicher Rückstand, der in 1 ml THF aufgenommen und für die quantitative Bestimmung durch kalibrierte HPLC verwendet wurde.

### Quantifizierung der Q10-Produktion durch kalibrierte HPLC:

Die Quantifizierung von Q10-haltigen Proben erfolgte durch kalibrierte HPLC. Verwendet wurde eine Agilent 1100 HPLC, ausgerüstet mit einer Elite Hy-Purity C18 Säule (Hersteller Phenomenex). Das Laufmittel (isokratisch) war zusammengesetzt aus 71 % (v/v) Acetonitril, 22 % (v/v) Dichlormethan, 4 % (v/v) Methanol, 2 % H₂O, und 1 % (v/v) Propionsäure. Die Flussgeschwindigkeit betrug 2 ml/min. Säulentemperatur war Raumtemperatur. Das Auftragsvolumen betrug 5 µl. Detektor war ein HP Diodenarraydetektor (Hewlett Packard), kombiniert mit einem Integrator zur Quantifizierung der Peakfläche. Gemessen wurde bei 280 nm Wellenlänge. Zur Kalibrierung wurde eine 0,2 mg/ml Standardlösung von Q10 (Sigma) in THF verwendet. Die Retentionszeit von Q10 unter den angegebenen Bedingungen betrug 10,3 min. Das UV-Spektrum des Peaks diente zur Identifizierung und Reinheitsbestimmung der Produktpeaks in zu quantifizierenden Proben. Aus dem Vergleich der Peakfläche der Q10-Standardlösung zur Q10-Peakfläche unbekannter Proben wurde die Q10-Menge quantitativ bestimmt. Aus der quantitativen HPLC Bestimmung wurde die Q10-Volumenproduktion (mg Q10/1 Anzuchtsmedium) und die Q10-Produktivität (mg Q10/g Trockenbiomasse) bestimmt.

### 2. Beispiel:

### Mutagenese von Sporidiobolus ruineniae mit UV-Strahlung oder N-Methyl-N'-Nitro-N-Nitrosoguanidin (NG)

### UV-Mutagenese auf YPD-Platten:

Sporidiobolus ruineniae Sr-1 wurde in einer Schüttelkolbenanzucht in YPD-Medium (1 % Hefeextrakt, 2 % Pepton, 2 % Glucose) 48 h bei 28°C kultiviert. Danach wurde die Kultur auf eine optische Dichte OD600 von 0,1 verdünnt und davon 0,1 ml auf YPD-Agarplatten (1 % Hefeextrakt, 2 % Pepton, 2 % Glucose, 1,5 % Agar) ausgestrichen. Danach wurde die Platte UV-Licht ausgesetzt (UV Lichtquelle: UV-Schirm TFP 20 M, 6 x 15 W, 213 nm von Firma Vilbert Lourmat, Frankreich). In Vorversuchen wurde die Strahlendosis (Expositionszeit und -intensität) bestimmt, die zur gewünschten Abtötungsrate der Sporidiobolus ruineniae-Zellen führte. Die Abtötungsrate sollte zwischen 50% und 99% betragen. Auf diese Weise UV-mutagenisierte Zellen wurden 2-5 Tage bei 28°C inkubiert, bis Kolonien sichtbar wurden. In parallelen Ansätzen wurden YPD-Platten mit Zusatzen verwendet, welche einen Selektionsdruck auf Sporidiobolus ruineniae ausübten und Q10-überproduzierenden Mutanten einen Wachstumsvorteil verschafften (siehe Beispiele 3 und 4).

### UV-Mutagenese in Zellsuspension

Sporidiobolus ruineniae Sr-1 wurde in einer Schüttelkolbenanzucht in YPD-Medium (1 % Hefeextrakt, 2 % Pepton, 2 % Glucose) 48 h bei 28°C kultiviert. Mit dieser Vorkultur wurde frisches YPD-Medium beimpft und unter gleichen Bedingungen weiter kultiviert, bis die S. ruineniae Zellen eine OD₆₀₀ von 0,4 erreichten. Von dieser Zellsuspension wurden 0,5 ml abzentrifugiert, in 2 ml 0,9% NaCl-Lösung resuspendiert und in einer sterilen Petrischale 20, 30 und 40 Sekunden einer UV-Behandlung ausgesetzt (UV Lichtquelle: UV-Schirm TFP 20 M, 6 x 15 W, 213 nm von Firma Vilbert Lourmat, Frankreich). Die mutagenisierten Zellen wurden anschließend auf YPD-Platten ausgebracht und 2-5 Tage bei 28°C inkubiert, bis Kolonien sichtbar wurden. In parallelen Ansätzen wurden YPD-Platten mit Zusätzen verwendet, welche einen Selektionsdruck auf Sporidiobolus ruineniae ausübten und Q10-überproduzierenden Mutanten einen Wachstumsvorteil verschafften (siehe Beispiele 3 und 4).

### NG-Mutagenese

Sporidiobolus ruineniae Sr-1 wurde in einer Schüttelkolbenanzucht in YPD-Medium (1 % Hefeextrakt, 2 % Pepton, 2 % Glucose) 48 h bei 28°C kultiviert. Mit dieser Vorkultur wurde frisches YPD-Medium beimpft und unter gleichen Bedingungen weiter kultiviert, bis die S. ruineniae Zellen eine OD₆₀₀ von 1,0 erreichten. Von dieser Zellsuspension wurden 10 ml abzentrifugiert, in 10 ml Citrat-Puffer (0,1 M Na-Citrat, pH 5,5) gewaschen und in 1 ml-Portionen in Citrat-Puffer mit verschiedenen Mengen an NG (0,01 bis 0,06 mg/ml) eine Stunde im Reagenzglas bei 28°C inkubiert. Danach wurden die Zellen abzentrifugiert, in 1 ml Phosphat-Puffer (0,1 M KH₂PO₄, pH 7,0 mit NaOH eingestellt) gewaschen und in verschiedenen Verdünnungen auf YPD-Platten aufgebracht. Die Platten wurden 2-5 Tage bei 28°C inkubiert, bis Kolonien sichtbar wurden. In parallelen Ansätzen wurden YPD-Platten mit Zusätzen verwendet, welche einen Selektionsdruck auf Sporidiobolus ruineniae ausübten und Q10-überproduzierenden Mutanten einen Wachstumsvorteil verschafften (siehe Beispiele 3 und 4).

Die hier beschriebenen Methoden sind dafür geeignet, Mutanten von Sporidiobolus ruineniae mit verbesserter Q10-Produktion zu isolieren. Dieses Verfahren ist insbesondere dazu geeignet, durch wiederholte Runden von Mutagenese und Selektion, bei der eine Mutante mit verbesserter Q10-Produktion ausgewählt und als Ausgangsstamm für die nächste Runde der Mutagenese und Selektion verwendet wird, die Q10-Produktion von S. ruineniae in deutlichem Ausmaß zu verbessern (siehe folgende Beispiele).

### 3.Beispiel:

### Selektion von Sporidiobolus ruineniae Mutanten mit verbesserter Q10-Produktion

Mutanten von S. ruineniae wurden hergestellt wie im 2. Beispiel beschrieben und unter selektiven Bedingungen kultiviert. Die selektiven Wachstumsbedingungen wurden so gewählt, dass bevorzugt solche Mutanten isoliert werden konnten, die aufgrund einer Mutation im Q10-Stoffwechsel zu einer erhöhten Q10 Produktion befähigt sein sollten.

Selektive Agenzien wurden in Konzentrationen eingesetzt, die das Wachstum des für die Mutagenese verwendeten Ausgangsstammes um 50-90 % reduzierten. Die selektiven Agenzien wurden, wenn nicht anders beschrieben, den YPD-Platten beigemischt, auf die die wie im 2. Beispiel hergestellten mutagenisierten Zellen ausplattiert wurden.

Im einzelnen wurden folgende selektiven Bedingungen gewählt: Paraquat wurde in Konzentrationen von 0,1-0,5% (w/v) den YPD-Platten zugesetzt. Menadion (2-Methyl-1,4-Naphthochinon) wurde in einer Konzentration von 3 µg/ml, 20 µg/ml, 50 µg/ml oder 100 µg/ml den YPD-Platten zugesetzt. Antimycin wurde den YPD-Platten in Konzentrationen zwischen 5 und 100 µM, Piericidin A in einer Konzentration von 20, 50 oder 100 µM beigesetzt.

### 4. Beispiel:

### Selektion von Sporidiobolus ruineniae-Mutanten durch Kombinationen verschiedener Zusätze

Mutagenisierte Sporidiobolus ruineniae-Zellen mit verbesserter Q10-Produktion wurden auch durch eine Kombination von verschiedenen selektiven Agenzien isoliert.

### Selektion mit einer Kombination von Paraquat und Linolensäure:

S. ruineniae Sr-1 Zellen wurden durch NG-Behandlung mutagenisiert wie im 2. Beispiel beschrieben und dann für 4 h bei 28°C in Schüttelkultur unter selektiven Bedingungen weiter inkubiert. Für die Schüttelkultur wurde YPD-Medium unter Zusatz einer Kombination der selektiven Agentien Paraquat, in einer Konzentration von 0,01 % und Linolensäure, in einer Konzentration von 50 mM oder 100 mM, eingesetzt. Nach der Inkubation für 4 h bei 28°C wurden die Zellen auf YPD-Platten ausplattiert.

### Selektion mit einer Kombination von Paraquat, Linolensäure und Piericidin A:

S. ruineniae Sr-1 Zellen wurden durch NG-Behandlung mutagenisiert wie im 2. Beispiel beschrieben und dann für 4 h bei 28°C in Schüttelkultur unter selektiven Bedingungen weiter inkubiert. Für die Schüttelkultur wurde YPD-Medium unter Zusatz einer Kombination der selektiven Agentien Paraquat, in einer Konzentration von 0,01 % und Linolensäure, in einer Konzentration von 50 mM oder 100 mM, eingesetzt. Nach der Inkubation für 4 h bei 28°C wurden die Zellen auf YPD-Platten ausplattiert, die zusätzlich Piericidin A in einer Konzentration von 50 µM enthielten.

YPD-Platten, die mit den auf die beschriebene Art und Weise vorbehandelten S. ruineniae Zellen beimpft worden waren, wurden 2-5 d bei 28°C bebrütet. Einzelkolonien der auf diese Weise hergestellter Mutanten von S. ruineniae wurden gepickt und auf frische YPD-Platten vereinzelt und erneut bebrütet. In jeder Mutageneserunde wurden so 400 Mutanten isoliert, welche anschließend auf eine gesteigerte Q10-Produktion hin untersucht wurden (siehe 5. Beispiel).

### 5. Beispiel:

### Analyse der Q10-Produktion von Sporidiobolus ruineniae Mutanten

Durch die in Beispiel 2 beschriebenen Mutagenese- und den in den Beispielen 3 und 4 beschriebenen Selektionsverfahren wurden Mutanten von Sporidiobolus ruineniae isoliert. Es wurden zwei Screeningrunden durchgeführt. In jeder Screeningrunde wurden 400 Mutanten gepickt und auf YPD Platten für die nachfolgende Q10-Analyse kultiviert. Die Q10-Analyse erfolgte durch Anzucht der Mutanten im Schüttelkolben und anschließender quantitativer Bestimmung der Q10-Produktion.

50 ml YPGC-Medium (1 % Yeast Extract, 2 % Pepton, 5 % Glycerin, 2 % Corn Steep Liquor) wurden jeweils mit einer Mutante beimpft und 120 h bei 28°C und einer Schüttlergeschwindigkeit von 140 rpm inkubiert. Danach wurden die Zellen abzentrifugiert, in 30 ml vollentsalztem Wasser gewaschen, in 5 ml vollentsalztem Wasser resuspendiert und in einen tarierten 50 ml Rundkolben überführt. Die Zellsuspension wurde anschließend in einer Ethanol/Trockeneis-Mischung möglichst gleichmäßig eingefroren und lyophilisiert. Die Auswaage der lyophilisierten Biomasse wurde als Trockenmasse bestimmt. 200 mg der Trockenmasse wurden ausgewogen, mit 10 ml Ethanol versetzt und mit gasförmigem Stickstoff überlagert. Die Q10-Extraktion erfolgte wie im 1. Beispiel beschrieben (Q10-Isolierung ohne mechanischen Aufschluss). Bestimmt wurde die Q10-Volumenproduktion (mg Q10/l) und die spezifische Q10-Produktion (mg Q10/g Biomasse). In jeder Screeningserie wurde der für die Mutagenese verwendete Ausgangsstamm als Referenz verwendet. Aus jeweils 400 Mutanten wurde durch Wiederholungsversuche der Stamm mit der am stärksten verbesserten Q10-Volumenproduktion ausgewählt und für die nächste Screeningrunde als Ausgangsstamm eingesetzt.

Tabelle 1 zeigt die Q10-Produktion einer Auswahl von Mutanten aus der ersten Screeningrunde, die wie in den Beispielen 1, 2 und 3 beschrieben hergestellt worden waren und sich gegenüber dem Ausgangsstamm durch eine deutlich verbesserte Q10-Produktion auszeichneten. Die Mutagenese von S. ruineniae erfolgte in der ersten Screeningrunde durch UV-Mutagenese.

**Tabelle 1:**

| Sporidiobolus ruineniae Sr-1 (DSM 15553) und Mutanten mit verbesserter Q10-Produktion. | | | |
|---|---|---|---|
| Stamm | Selektion | Q10-Produktion (mg/g) | Q10-Produktion (mg/l) |
| Sr-1 | - | 0,95 | 8,2 |
| Sr-5 | 0,1 % Paraquat | 1,45 | 10,8 |
| Sr-15 | 0,1 % Paraquat | 2,20 | 13,5 |
| Sr-43 | 0,1 mM Menadion | 1,51 | 15,7 |
| Sr-61 | 0,1 mM Menadion | 1,81 | 13,2 |
| Sr-176 | 0,1 mM Antimycin | 1,66 | 10,3 |
| Sr-178 | 0,1 mM Antimycin | 1,59 | 11,0 |
| Sr-209 | 0,01 % Linolensäure | 1,66 | 13,6 |
| Sr-220 | 0,01 % Linolensäure | 2,10 | 17,8 |

Aus der ersten Screeningrunde wurde Stamm Sr-220 für die zweite Screeningrunde ausgewählt. In der zweiten Screeningrunde wurden Mutanten des Stammes Sr-220 durch NG-Mutagenese erzeugt. Die Selektion der Mutanten erfolgte durch eine Kombination von Paraquat und Linolensäure. Eine Auswahl in ihrer Q10-Produktion stark verbesserter Mutanten ist in Tabelle 2 aufgeführt.

**Tabelle 2:**

| Sporidiobolus ruineniae Stamm Sr220 und Mutanten mit verbesserter Q10-Produktion. | | | |
|---|---|---|---|
| Stamm | Selektion | Q10-Produktion (mg/g) | Q10-Produktion (mg/l) |
| Sr220 | - | 2,18 | 18,1 |
| Sr220-87 | 0,1 % Paraquat + 0,01 % Linolensäure | 2,34 | 19,3 |
| Sr220-121 | 0,1 % Paraquat + 0,01 % Linolensäure | 3,50 | 22,9 |
| Sr220-148 | 0,1 % Paraquat + 0,01 % Linolensäure | 2,66 | 20,9 |
| Sr220-159 | 0,1 % Paraquat + 0,01 % Linolensäure | 2,94 | 24,8 |
| Sr220-218 | 0,1 % Paraquat + 0,01 % Linolensäure | 2,29 | 19,7 |
| Sr220-304 | 0,1 % Paraquat + 0,01 % Linolensäure | 2,71 | 21,8 |
| Sr220-307 | 0,1 % Paraquat + 0,01 % Linolensäure | 2,57 | 20,2 |

Aus der zweiten Screeningrunde wurde der Stamm Sr220-159 ausgewählt, um ein Verfahren zur Fermentation von S. ruineniae zu entwickeln (siehe 6. und 7. Beispiel). Ein Vergleich des S. ruineniae Wildtypstammes Sr-1 und der Mutante Sr220-159 zeigt, dass durch das neuartige Stammverbesserungsverfahren die Q10-Produktion von S. ruineniae signifikant verbessert werden kann. Durch zwei Runden von Mutagenese und Selektion wurde die Q10-Produktivität (mg Q10/g Biomasse) und die Volumenproduktion (mg Q10/1 Anzuchtsmedium) um jeweils ca. 300 % erhöht.

### 6. Beispiel:

### Batch Fermentation von S. ruineniae

Für die Batch Fermentation wurde ein Fermenter Biostat M (Braun, Melsungen) verwendet. Das Kulturgefäß hatte ein Gesamtvolumen von 2 1. Das Arbeitsvolumen betrug 1 1. Der Fermenter war darüber hinaus mit Elektroden zur Messung des pO₂ und des pH ausgestattet. Der pH der Fermentation betrug 6,0 und wurde mit Ammoniumhydroxid und Phosphorsäure geregelt. Druckluft wurde in das Fermentationsgefäss mit 1,6 1/min eingeleitet. Die Fermentationstemperatur betrug 28°C, die Rührgeschwindigkeit 900 rpm. Der Fermenter war darüber hinaus mit einer Schaumsonde ausgerüstet, welche die Zudosierung von Antischaum Structol J 647 (Schill und Seilacher), 1:5 mit H₂O verdünnt, regulierte. Der Fermenter wurde mit 1 1 sterilem YPGC-Medium (siehe 1. Beispiel) beschickt. Zur Erzeugung eines Inokulums wurde eine erste Vorkultur des Stammes Sporidiobolus ruineniae Sr220-159 in gleichem Medium für vier Tage, 28°C, 140 rpm (Schüttler, Infors) hergestellt. 5 ml der ersten Vorkultur wurde zum Beimpfen einer zweiten Vorkultur in 50 ml YPGC-Medium verwendet. Die zweite Vorkultur wurde über Nacht bei 28°C und 140 rpm geschüttelt. Mit der zweiten Vorkultur wurde der Fermenter steril beimpft und die Fermentation gestartet. Die Fermentationsdauer betrug 96 h. Nach Beendigung der Fermentation wurden die Trockenbiomasse und Q10 bestimmt, wie im 1. Beispiel beschrieben. Die Trockenbiomasse betrug 25 g/l und die spezifische Q10-Ausbeute betrug 0,89 mg Q10/g Trockenbiomasse.

### 7. Beispiel:

### Fed-Batch Fermentation von S. ruineniae

Für die Fed-Batch Fermentation wurde ein Fermenter Biostat M (Braun, Melsungen) verwendet. Das Kulturgefäß hatte ein Gesamtvolumen von 2 1. Das Arbeitsvolumen betrug 1 1. Der Fermenter war darüber hinaus mit Elektroden zur Messung des pO₂ und des pH ausgestattet. Der pH der Fermentation betrug 6,0 und wurde mit Ammoniumhydroxid und Phosphorsäure geregelt. Druckluft wurde in das Fermentationsgefäß mit 1,6 1/min eingeleitet. Die Fermentationstemperatur betrug 28°C, die Rührgeschwindigkeit 900 rpm. Der Fermenter war darüber hinaus mit einer Schaumsonde ausgerüstet, welche die Zudosierung von Antischaum Structol J 647 (Schill und Seilacher), 1:5 mit H₂O verdünnt, regulierte.

Der Fermenter wurde mit 1 1 sterilem YPGC-Medium (siehe 1. Beispiel) beschickt. Zur Erzeugung eines Inokulums wurde eine erste Vorkultur des Stammes Sporidiobolus ruineniae Sr220-159 in gleichem Medium für vier Tage, 28°C, 140 rpm (Schüttler, Infors) hergestellt. 5 ml der ersten Vorkultur wurde zum Beimpfen einer zweiten Vorkultur in 50 ml YPGC-Medium verwendet. Die zweite Vorkultur wurde über Nacht bei 28°C und 140 rpm geschüttelt. Mit der zweiten Vorkultur wurde der Fermenter steril beimpft und die Fermentation gestartet. Die Fermentationsdauer betrug 96 h. 24 h nach Beginn der Fermentation wurde die Fütterung gestartet. Die Fütterungslösung bestand aus 75 % (v/v) Glycerin, 2 % (w/v) Hefeextrakt. Die Fütterungsgeschwindigkeit betrug von 24 h bis 48 h der Fermentation 12 ml/h, von 48 h bis zum Ende der Fermentation (96 h) 6 ml/h. Zu verschiedenen Zeiten wurde Fermentermedium gewonnen für die quantitative Bestimmung der Q10-Ausbeute. Die Q10-Bestimmung erfolgte nach der Dismembrator Methode (siehe 1. Beispiel). Zum Ende der Fermentation betrug die Biomasse 73,6 g/l und die Q10-Ausbeute betrug 3,12 mg/g Trockenbiomasse. Dies entspricht einer signifikanten Steigerung gegenüber Batchfermentationen und dem Stand der Technik für Sporidiobolus ruineniae (US 4070244, Q10-Produktion 0.92 mg/g, Biomasse 18 g/l).

## Patentansprüche

1. Verfahren zur Herstellung eines mutagenisierten Sporidiobolus ruineniae Stammes mit einer Q10-Produktivität von großer 1,38 mg Q10/g Trockenbiomasse **dadurch gekennzeichnet, dass**
a) ein Sporidiobolus ruineniae Stamm einer Genveränderung durch Mutagenese ausgesetzt wird und
b) der mutagenisierte Sporidiobolus ruineniae Stamm einer Selektion unterworfen wird, wobei der mutagenisierte Sporidiobolus ruineniae Stamm unter Bedingungen, die ein Wachstum des in a) eingesetzten Sporidiobolus ruineniae Stammes hemmen, kultiviert wird, wobei die Bedingungen so gewählt sind, dass der mutagenisierte Stamm durch eine im Vergleich zu dem in a) eingesetzten Sporidiobolus ruineniae Stamm erhöhte Q10-Produktion die Wachstumshemmung überwindet, in einem Fermentationsmedium wächst und
c) aus dem Fermentationsmedium isoliert wird.

2. Verfahren gemäß Anspruch 1, **dadurch gekennzeichnet, dass** die Mutagenese durch Einwirkung mutagener Substanzen oder hochenergetischer Strahlung auf das S. ruineniae Genom bewirkt wird und bei der Mutagenese eine Abtötungsrate von 30 bis 99 % bewirkt wird.

3. Verfahren gemäß Anspruch 1 oder 2, **dadurch gekennzeichnet, dass** die Selektion des Sporidiobolus ruineniae Stammes mit einer Q10-Produktivität von größer 1,38 mg Q10/g Trockenbiomasse durch Bedingungen, die einen oxidativen Stress erzeugen, erfolgt.

4. Verfahren gemäß Anspruch 3, **dadurch gekennzeichnet, dass** der oxidative Stress durch Paraquat, Wasserstoffperoxid oder ungesättigte Fettsäuren wie Linolensäure erzeugt wird.

5. Verfahren gemäß einem der Ansprüche 1 oder 2, **dadurch gekennzeichnet, dass** die Selektion des Sporidiobolus ruineniae Stammes mit einer Q10-Produktivität von größer 1,38 mg Q10/g Trockenbiomasse durch einen Inhibitor der Atmungskette erfolgt.

6. Verfahren gemäß Anspruch 5, **dadurch gekennzeichnet, dass** der Inhibitor der Atmungskette Antimycin A, Piericidin, Mucidin, Rotenon oder Menadion ist.

7. Verfahren gemäß Anspruch 1 oder 2, **dadurch gekennzeichnet, dass** die Selektion des Sporidiobolus ruineniae Stammes mit einer Q10-Produktivität von größer 1,38 mg Q10/g Trockenbiomasse durch eine Hemmung eines Schrittes des Q10-Biosyntheseweges von Sporidiobolus ruineniae erfolgt.

8. Verfahren gemäß Anspruch 7, **dadurch gekennzeichnet, dass** die Hemmung eines Schrittes des Q10-Biosyntheseweges von Sporidiobolus ruineniae mittels Glyphosat, Lovastatin, Cerivastatin, Atorvastatin, Compactin, oder Ethionin erfolgt.

9. Mutierter Sporidiobolus ruineniae Stamm erhältlich mittels eines Verfahrens gemäß einem oder mehreren der Ansprüche 1 bis 8, **dadurch gekennzeichnet, dass** er eine Q10-Produktivität von mehr als 1,38 mg Q10/g Biomasse besitzt.

10. Verfahren zur Produktion von Q10, **dadurch gekennzeichnet, dass** Zellen eines Stammes gemäß Anspruch 9 in einem Anzuchtmedium kultiviert werden, die Zellen nach einer Wachstumszeit isoliert werden und daraus Q10 isoliert wird.

11. Verfahren gemäß Anspruch 10, **dadurch gekennzeichnet, dass** eine Anzucht der Zellen im Fed-Batch Modus erfolgt, wobei nach einer anfänglichen Wachstumsphase im Batch Modus in einem Feed zusätzlich Nährstoffquellen zugefüttert werden um deren Verbrauch auszugleichen.

12. Verfahren gemäß Anspruch 11, **dadurch gekennzeichnet, dass** die Nährstoffquellen im Feed eine C-Quelle, eine N-Quelle oder ein Gemisch der beiden umfassen.

13. Verfahren gemäß Anspruch 12, **dadurch gekennzeichnet, dass** die C-Quelle im Feed ausgewählt ist aus Glycerin, Glucose, Saccharose, Melasse, Maltose oder Acetat und die N-Quelle ausgewählt ist aus Ammoniak bzw. Ammoniumsalzen, Hefeextrakt, Sojapepton, Malzextrakt oder Corn Steep liquor.
